# EUROPEAN PATENT APPLICATION

(11) **EP 4 787 383 A2**
(43) Date of publication of application: **05.08.2026**
(21) Application number: 26187120.6
(22) Date of filing: 08.02.2024
(51) Int. Cl.: G16H 20/13

(54) **PHARMACEUTICAL DISPENSING DEVICE**

(30) Priority: 09.02.2023 NL 2034123
(62) Divisional of application: 24704927.3
(71) Applicant: ReMediZ B.V., 4811 XH Breda (NL)
(72) Inventor: DECKERS, Johannes Henricus Petrus, 4811 XH Breda (NL)
(74) Representative: V.O.

(57) **Abstract**

The present disclosure pertains to a pharmaceutical product (200) comprising a carrier card (210) with a support portion (211) supporting at least one singulated unit dose blister package (100) and a data portion (212) with regulatory data. The carrier card (210) comprises opposing side border portions configured to engage with a receptacle (10) of a pharmaceutical dispensing device, and a bottom end face configured to engage with an ejector element (61) of the pharmaceutical dispensing device. The blister package (100) is held securely between the support portion (211) and a cap (220). Both the support portion and the cap have an aperture (214, 224) allowing access to the blister by pressing on a top film of the blister package (100) to break the bottom seal for releasing the medicament while the unit dose blister package (100) is held securely between the support portion (211) and the cap (220).

## Description

### TECHNICAL FIELD AND BACKGROUND

The present disclosure relates to a pharmaceutical product comprising a singulated unit dose package. The pharmaceutical product is configured to be dispensed by a device and method as described herein.

In a pharmacy the process of issuing medication involves several steps to ensure that the medication is issued in a correct, safe and timely manner. In a prescription verification step the pharmacist will verify the prescription as provided by a healthcare provider to ensure that it is valid and that the medication prescribed is appropriate for the patient. In a preparation step the medication will be prepared for issuing, e.g. to a patient directly of to an authorized care professional, in a batch comprising dosages covering a prescribed period.

Typically, however, not all medication is used. As a result, medication is often left over, which is generally destroyed or otherwise disposed of and thereby no longer available for later reissuing. Additionally there can be lack of monitoring compliance of medication intake. Accordingly, there remains a need to means and methods that improve on the effectivity of medication distribution and/or traceability. Improving effectivity of the dispensing and/or traceability of medication can be especially relevant for medication that is costly and/or in scarce supply.

### SUMMARY

Aspects of the present disclosure relate to a pharmaceutical product as defined in the appended claims.

As background, the present disclosure further describes a pharmaceutical dispensing device, and a method of dispensing the pharmaceutical product.

The device comprises at least: a secure access storage compartment configured for housing at least one receptacle for holding in storage a pharmaceutical product to be dispensed; means for displacing the pharmaceutical product from the receptacle to an outlet for dispensing; and means for logging storage parameters including storage compartment temperature readings. Advantageously the device can be a compact portable device for dispensing pharmaceuticals in a vicinity of a patient location, including but not limited to: at home, near a patient bed, or a hospital.

Storing the pharmaceutical product and logging storage parameters including storage compartment temperature readings at least until dispensing realizes traceability of storing conditions that can allow restocking of unspent medication for reissuing. As will become clear from the specification herein the device according the invention can advantageously be understood as an extension of an issuing facility, e.g. a pharmacy, such that medication stored within the device can, for regulatory purposes, be understood as having never left a regulatory storage environment. In this way the invention can contribute to mitigating wastage or medication as compared to conventionally issued medication procures that incompatible with re-stockage procedures.

In general terms, the pharmaceutical product can be understood as a single or singulated package comprising a unit dose of a prescription medicament. Preferably, the pharmaceutical product comprises a singulated unit dose blister package. Multi dose blister packages, e.g. blisters packages comprising plurality of doses, are widely used in for packaging products such as pills or capsules. Such packages can start a basis for singulation, resulting in the provision of individualized packages comprising one or a more limited number of doses, e.g. two, using known technology in such a way that the original seal (between the top and a usually aluminum foil bottom) is maintained. The existing medicine therefore does not comes into a direct contact with ambient prior to dispensing by the device.

In a preferred embodiment the pharmaceutical product comprises a carrier card having a support portion supporting at least one singulated unit dose blister package or an alternative single unit dose packaged medicament. A will be clear from the description herein pharmaceutical products supported by a carrier card can be particularly effectively stored and dispensed by the device according to the invention. Generally, the carrier card will also comprise a data portion with regulatory information regarding the unit dose packaged medicament. The card may also be provided with appropriate user instructions. Optionally, the card may comprise further data, including but not limited to a barcode, QR-code or another type ID known in the field, such as an RF-ID.

In a particularly preferred embodiment the singulated unit dose blister package is supported between the support portion and a cap, said cap being affixed to the support portion from a base around a perimeter of the unit dose blister package, wherein both the support portion and the cap have an aperture allowing access to the blister. Provision of the holes advantageously allows an end-user (e.g. a patient) to retrieve the packaged dose in an essentially unhindered manner by pressing on a top film of the blister to break the bottom seal for releasing the medicament.

In some embodiments, the pharmaceutical product, e.g. the singulated unit dose blister package, comprises two or more packaged unit doses. For example, the carrier car can support two (or more) singulated blisters packages, each with a single packaged unit does in a side by side relation. Alternatively, or in addition, the singulated blister portion can comprise two (or more) packaged unit doses. It will be appreciated that the carrier can also comprise other variations of packaged unit doses, such as combinations of the embodiments listed above. Products comprising multiple unit dose packages advantageously allows dispensing of multiple medicaments in a single operation, which can assist patient compliance as regard to a prescription specifying intake of multiple doses of a single medicament or a combination of medicaments at a time.

The invention can also be worked on other packaged pharmaceuticals, including individualized packaged pharmaceuticals, such as a prefilled syringe, a bottle, a sachet, cases, and the like.

In a preferred embodiment, the pharmaceutical dispensing device, comprises: a main body case comprising a secure access storage compartment; a conveyor housed in the secure access storage compartment, the conveyor comprising at least one receptacle for holding in storage a pharmaceutical product; a positioning system acting on the conveyor and configured to position the receptacle to a dispensing position within the storage compartment, whereby the pharmaceutical product is positioned between an ejector and a dispensing outlet; and a control system operably connected to the positioning system and the ejector and configured for logging storage parameters including at least temperature readings from a temperature sensor housed within the secure access storage compartment, wherein the pharmaceutical product comprises a carrier card having a support portion supporting at least one singulated unit dose blister package and a data portion, wherein the dispensing aperture opens at a top portion of the secured access storage compartment above the dispensing position, and wherein the ejector comprises an ejector element that is reversibly displaceable along a trajectory between a rest position below the dispensing position when idle and an extended position toward the outlet aperture when activated and configured to engage a bottom end face of the carrier card and thereby push the pharmaceutical product upward from the receptacle and at least partly or entirely out of the dispensing outlet for dispensing.

Preferably, the controller is further configured to log a storage state of the receptacles, including one or more, preferably all, of: a type and quantity of a medicament as provided to each of the one or more receptacles, a time of dispensing, an order of loading and/or unloading of the receptacles, and readings of one or more environmental conditions inside and/or outside the device.

The device as specified can advantageously provide traceability of pharmaceutical products under secure storage conditions and controlled dispensing. This contributes to patient compliance of prescription medication and enables regulatory restocking of pharmaceuticals, provided logged data is within spec.

Dispensing the pharmaceutical product from a top portion of the compartment, e.g. via an aperture in a secured cover lid, advantageously reduces disturbances as to the storage conditions within the storage volume, e.g. in terms of variations in temperature and/or humidity due to air exchange as a result of a dispensing operation.

In a preferred embodiment, the conveyor is formed as a carrousel comprising a plurality of the receptacles at intervals along the conveyor, the carrousel being rotatable around one or more upstanding axles extending from a base of the secure storage compartment toward the top portion, each receptacle configured for holding in storage at least one of the pharmaceutical product in an upright orientation. Arranging the receptacles in an carrousel (an endless conveyor) advantageously increases a loading capacity within the housing as compared to a reciprocal conveyor. The receptacles can be removably connected links along a belt forming a flexible endless conveyor. Arranging the carrousel around two (or more) upstanding axels, at least one of which can be driven, can advantageously realize a space within the compartment, for housing one or more sensors.

The receptacle can comprise a guide structure which includes a pair of slots that extend opposingly along sides of the receptacle across a conveying direction of the conveyor forming a slide to receive the pharmaceutical product in an upright orientation by engaging with opposing side border portions of the carrier card. Storing the pharmaceutical products in an endless conveyor in combination with an upright orientation advantageously allows a comparatively high storage density (pharmaceuticals per unit volume) in combination with efficient dispensing with a single ejector.

In a further preferred embodiment a separation between a first one of the guide structures and a second one of the guide structures adjacent the first is less than two times a thickness of the pharmaceutical product and greater than two times a thickness of the carrier card (. In this way a dense packing within a link can be realized by inserting cards such that blister projections of singulated unit dose blister packages held by the adjacent first and second guide structures overlap.

In strongly preferred embodiments the main body case further comprises a temperature regulation acting on the secured access storage compartment. The temperature regulation being operable by the control system in dependence of at least a reading of the temperature sensor. In some embodiments the device comprises one or more additional temperature sensor within the storage volume to provide redundancy and/or to enable registration of a temperature distribution within storage. In other or further embodiments, the device comprises one or more additional temperature for registering ambient conditions (e.g. temperature outside the housing).

The temperature regulation preferably includes a Peltier element acting on the secure access storage compartment to provide cooling/heating as needed. Preferably, the Peltier element acts on a metal bound of the secure access storage compartment, preferably a bottom bound opposite the dispensing outlet.

The main electronic components, including temperature regulation, the controller, the ejector, and the positioning system, can advantageously be housed predominantly outside the secured access storage compartment. This improves serviceability and isolates the pharmaceuticals from main electronics further mitigating disturbances to the storage conditions. In a preferred embodiment, the main electronic components, including the controller, the ejector, and the positioning system are predominantly housed within one or more separate compartments of the main body case at a position below the secure access storage compartment. This can minimize complexity of a lid covering the main storage compartment and improves overall ease of manufacturing of the system.

The ejector element can, in principle be, any suitably shaped pusher, e.g. a rod or even an air flow, configured to displace the stored product from its storage position within the receptible for dispensing. Preferably, the ejector element comprises a resilient strip displaceable by a reel or by a rack-and-pinion assembly which is predominantly orientated a conveying direction of the conveyor. In this way the ejector can be accommodated in a compartment having a height less than an extended length the ejector element, contributing to overall compactness of the device.

To minimize access and air exchange between ambient and the storage volume the outlet can be designed in accordance with a projection of the pharmaceutical product(s) along the dispensing direction. Clearance be be ≤ 1mm. Alternatively, or in addition, it is foreseen to provide a barrier that extends across the dispensing outlet when idle and which allows passage of the pharmaceutical product during dispensing. In some embodiments the barrier comprises one or more of a door, a slitted membrane, opposing bristles, or the like.

In some embodiments, the device can further comprise one or more of: a humidity sensor and a radiation dosimeter operably connected to the controller for logging.

The method of dispensing a pharmaceutical product comprises the steps of i) providing a dispensing device comprising a secure access storage compartment housing at least one receptacle for holding in storage a pharmaceutical product to be dispensed, means for displacing the pharmaceutical product from one of the at least one receptacle to an outlet for dispensing, and means for logging storage parameters including storage compartment temperature readings, ii) loading one or more of the at least one receptacle with a pharmaceutical product to be dispensed in accordance with one or more instructions provided by a medical doctor, followed by iii) securing the secure access storage compartment prior to clearing the device for a dispensing of pharmaceutical products held in storage, iv) logging the storage parameters, over a period between a time of the clearing the device for the dispensing and a time of receiving the device back from the dispensing, v) restocking any of the pharmaceutical product remaining in storage provided the storage parameters are within tolerance limits for storing the pharmaceutical product, wherein steps ii), iii) and iv) are performed at a pharmacy or at least a facility operating within the tolerance limits for storing the pharmaceutical product.

The device advantageously allows on demand dispensing of medication, e.g. the pharmaceutical product as described herein, whereby temperature and optionally relative humidity and/or other environmental conditions, are not only logged but preferably also actively regulated within tolerances, and whereby a dispensing moment can be registered to support therapy compliance.

### BRIEF DESCRIPTION OF DRAWINGS

These and other features, aspects, and advantages of the pharmaceutical product of the present disclosure will become better understood from the following description, appended claims, and accompanying drawing wherein:
FIG 1 illustrates aspects of a singulated unit dose blister package;
FIGs 2A and 2B illustrate components for manufacturing of a pharmaceutical product comprising a singulated unit dose blister package;
FIG 3 illustrates a pharmaceutical product;
FIGs 4A to 4C illustrate views of a receptacle holding pharmaceutical products;
FIGs 5A to 5C illustrate views of a receptacle for pharmaceutical products;
FIG 6A and B illustrate aspects of carrousels comprising a plurality of the receptacles for a pharmaceutical dispensing device;
FIG 7 illustrates aspects of the carrousel;
FIG 8 illustrates an embodiment of a pharmaceutical dispensing device;
FIG 9 provides a cross-section side view of a pharmaceutical dispensing device;
FIGs 10 provides a cross-section side view of a pharmaceutical dispensing device;
FIGs 11A and 11B illustrate aspects of an ejector of a pharmaceutical dispensing device;
FIG 12 illustrates aspects of an ejector for a pharmaceutical dispensing device;
FIG 13 schematically illustrates a method of dispensing a pharmaceutical product; and
FIGs 14A-D illustrate several embodiments of a pharmaceutical product according to the invention.

### DESCRIPTION OF EMBODIMENTS

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise.

The invention as disclosed herein does not pertain to the act of, or the process leading up, providing a prescription by a medical specialist, e.g. a medical doctor.

Singulation as used herein can be understood as a process of separating individual packaged pharmaceutical products from a larger group or container, e.g. a multi blister package. It will be appreciated that singulation is nor restricted to blister packages. For example, singulation can be used to individualize one or more pre-packaged and pre-filled syringe or sachets from a multi-unit packaging.

Blister packaging is a method known for packaging pharmaceutical products such as tablets and capsules. Singulation of blister packaged products involves separating the individual blisters from a larger sheet. This process is usually done using a blister pick and place machine. The machine can use a combination of vacuum and mechanical manipulation to pick up and transfer the blisters to a conveyor belt or other equipment for cutting (or otherwise) into smaller portion having an appropriate number of blisters. US20110132163 discloses an apparatus for singulating unit dose blisters.

Restocking leftover medication after issuance (e.g. from a pharmacy) can be hindered by a number of factors including legal or regulatory requirements. A major hindrance is a lack of traceability after issuing. If medication is not stored or handled properly quality of the medicament may be at risk making it unsafe to restock.

The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

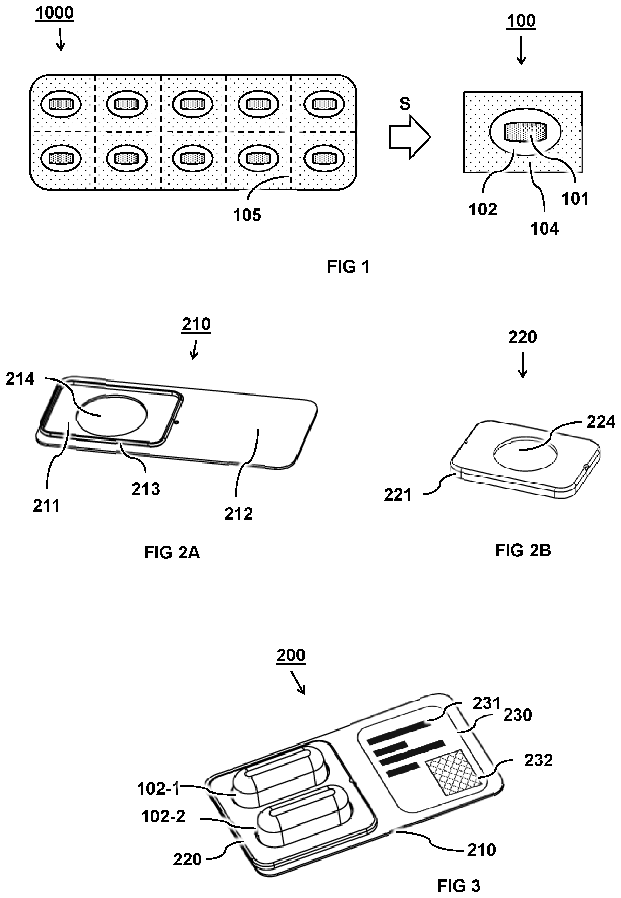

Figures 1 and 2 respectively illustrate aspects of a singulated unit dose package 100 and an exemplary pharmaceutical product 200 that can be used to advantage with the dispensing device as disclosed herein.

FIG 1 illustrates aspects of a singulated unit dose blister package 100 which has been singulated from a multi-blister package, which has been exemplified from a commercially available multi unit dose package 1000 (shown as a 2 × 5 array) in a way such that the original sealing protecting a packaged dose, e.g. pill 101, within cavity 102 on base portion 104 (also referred to as blister) is maintained. The process of singulating (S) itself is known in the field typically involves cutting the multi-unit dose package 1000 in to smaller pieces comprising a reduced number of doses, e.g. along cut lines marked 105. The singulated unit dose blister package 100 is, after an optional quality control, used provide the pharmaceutical product for individualized dispensing as disclosed herein.

FIGs 2A and 2B illustrate components for manufacturing of a pharmaceutical product comprising a singulated unit dose blister package. The pharmaceutical product comprises at least a carrier card 210 having a support portion 211 for supporting at least one singulated unit dose package and a data portion 212 for data regarding the unit dose package. The unit dose package can be assembled onto the card using known methods, e.g. gluing, tape or otherwise. In a preferred embodiment, the unit dose blister package is supported between the support portion and a cap 220, said cap being affixable to the support portion from a base, e.g. rim 221, around a perimeter of the unit dose blister package. Preferably, both the support portion and the cap have an aperture 214,224 allowing access to the blister. In this way the unit dose is can be held securely while providing access to the blister 102 for releasing medicament 101. In other words, the unit dose blister package is securely locked inside the pharmaceutical product, i.e. between the support portion and the cap. Accordingly, during use the pharmaceutical product and the unit dose blister package remain together as a single unit, e.g. while stored and handled by the dispensing device and also after being ejected by the dispensing device. In this way, the unit dose blister package and any information related thereto provided on the data portion 212 are coupled at all times, even when the medication is to be taken by the user. Additionally, the disclosed support and cap can advantageously allow packaging the blister portion without compromising the integrity of the blister. In a preferred embodiment, the cap and card can be assembled in a click or snap-on relation, e.g. by means of a ridge 213 provided onto the support region 211 of the card and a corresponding receptacle provided by the cap, e.g. along its the base 221. Alternatively, the cap and card can be coupled by welding, e.g. ultrasonic welding, of parts formed of the same or a similar thermoplastic polymer composition. Inventors find that ultrasonic welding can be performed without compromising the integrity of blister package.

FIG 3 illustrates an exemplary pharmaceutical product 200. In some embodiments, e.g. as shown, the carrier supports a dual singulated unit dose package. As indicated, the cover and carrier are provided with a corresponding apertures to provide direct access to blisters 102-1,102-2. As **indicated** a data sheet 230 can be provided onto the data portion 212 of the card, at the same side comprising regulatory information 231 pertaining to the packaged medication. Alternative ways to provide data include printing or laser scribing and the like. As shown, the product can include a further means for identifying the card, e.g. a machine readable tag 232 such as a QR code o RFID chip. The card can advantageously increase a flexural rigidity of the blister package, allowing the pharmaceutical product to be dispensed by exerting a force, preferably a mechanical force, on a terminal; side face of the card. The terminal end face can be determined by a thickness of the card. The card can be made of a solid composition, e.g. plastic or metal. The thickness is typically in a range of ≥ 0.1 mm and as thin as possible while allowing dispensing as disclosed herein. In some embodiments, the thickness is 0.4- 0.8 mm, e.g. about 0.6 mm.

The pharmaceutical product 200 can advantageously be provided in a standardized dimensions, e.g. in one, or a limited number, of standardized dimensions.

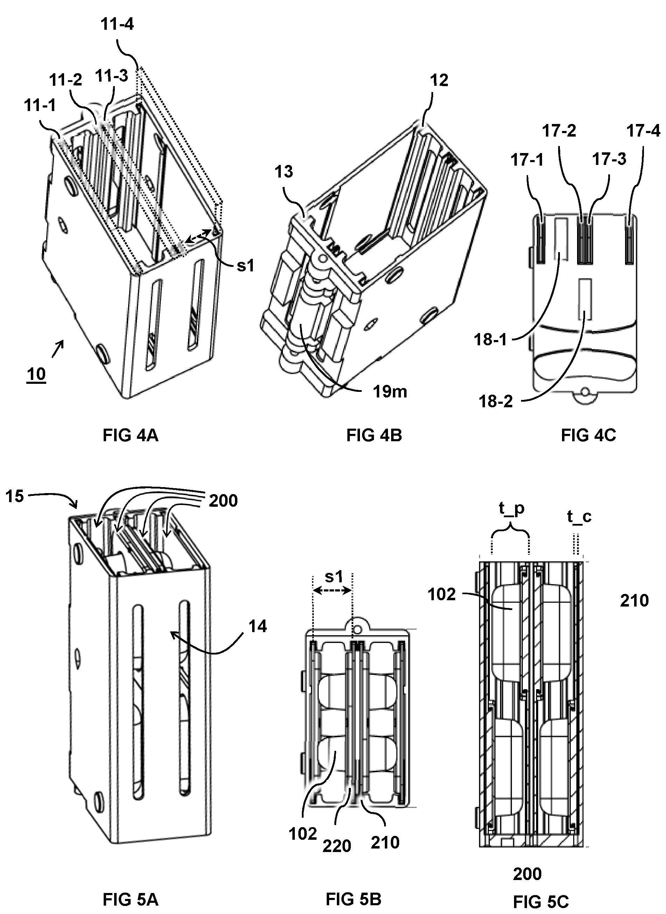

FIGs 4 and 5 illustrate aspects of an embodiment of a receptacle 10 for holding in storage pharmaceutical products to be dispensed. FIG 4A and 4B provide isometric views of an empty receptacle. FIG 4C provides a bottom view of the receptacle shown in FIGs 4A ad 4B. FIG 5A provides an isometric view of the receptacle shown in FIG 4 holding in storage a total of four dual pharmaceutical products. FIGs 5B and 5C provide corresponding top-down and a cross-section side views of the receptacle.

The link 10 is configured for holding in storage at least one of the pharmaceutical product 200 in an upright orientation. As illustrated, the receptacle can be embodied as a link which can be removably mounted (mount 19m) to a conveyor in a side-by-side fashion. The embodiment shown in FIGs 4 and 5 is configured to hold from 0 up to 4 pharmaceutical products when fully loaded. As best seen in FIGs 4A and 4B the receptacle comprises a guide structure for receiving the pharmaceutical product. Each guide structure 11-1, 11-2, 11-3, 11-4 comprises a pair of slots 12, 13 extending opposingly along sides 14,15 of the receptacle forming a slide to receive the pharmaceutical product in an upright orientation by engaging with opposing side border portions of the carrier card.

As best seen in the FIG 4C a bottom face of the receptible provides access to a terminal end face each of the carrier cards held in the guide structures. In the embodiment shown the bottom face is provided with a four apertures 17-1, 17-2, 17-3, 17-4. The apertures allow the ejector element (explained in more detail below) to engage the car to push the product in storage upward out of the receptacle. As shown the apertures can, but need not be, positioned directly centrally (mid) with regard to a position of the card because the guide structures enable smooth dispensing. directly below the carrier card.

In addition the receptacle can be provided with one or more positions for receiving alignment elements 18-1,18-2 to enable accurate positioning of each the receptacle by the conveyor with respect to the ejector. In some embodiments, e.g. as shown, the alignment element can be a reflector or a mirror. In some embodiments, the receptible comprises multiple alignments, e.g. a further element for identification of the receptacle and/or for defining a zero position of the receptacle. In some embodiments positioning can comprise activating a first stepper motor acting on the conveyor to position one of the links to the correct dispensing position, e.g. using the a gear reduction, a bearing main drive shaft, and a chain drum as described herein. This link can be selected, e.g. by the controller from logged store positions, to comprise a desired medicine to be dispensed. Positioning can comprise feedback control, e.g. based on optical positioning means.

As best seen in FIG 4A and 5B and 5C a separation (s1) between a first one of guide structures and a second one of the guide structures adjacent the first can advantageously be between two times a thickness (t_p) of the pharmaceutical product and two times a thickness (t_c) of the carrier card (T_c < s1 < t_p) such that projections of singulated unit dose blister packages held by the adjacent first and second guide structures overlap, e.g. by up and downward orientation of adjacent pharmaceutical products. It will be understood that the uppermost product will typically be dispensed first.

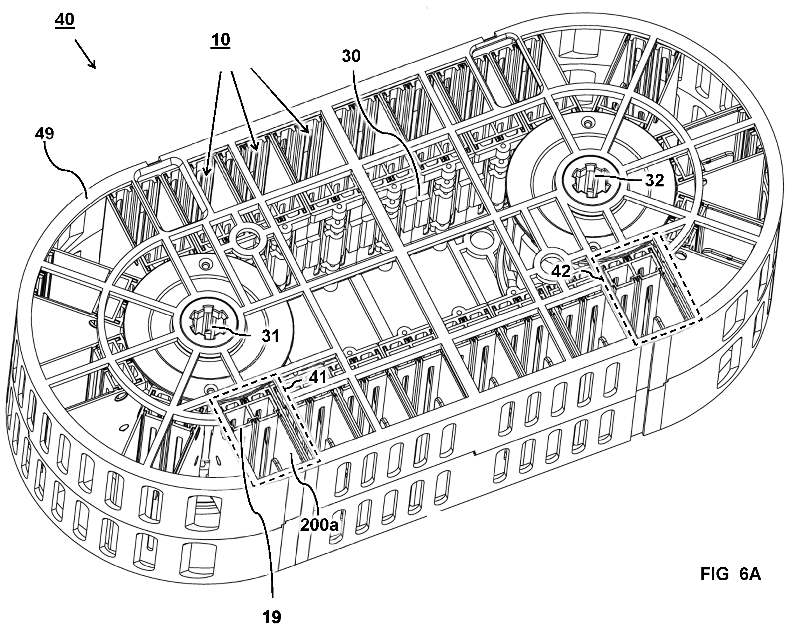

Fig 6A illustrates an embodiment wherein the conveyor 30 is formed as a carrousel 40 comprising a plurality of the receptacles 10 at intervals along the conveyor. In the embodiment shown the conveyor is provided with 22 links which can be conveyed around two axles 31,32 along an endless loop. Note that in the embodiment as shown each link is configured to receive up to four of the pharmaceutical products described.

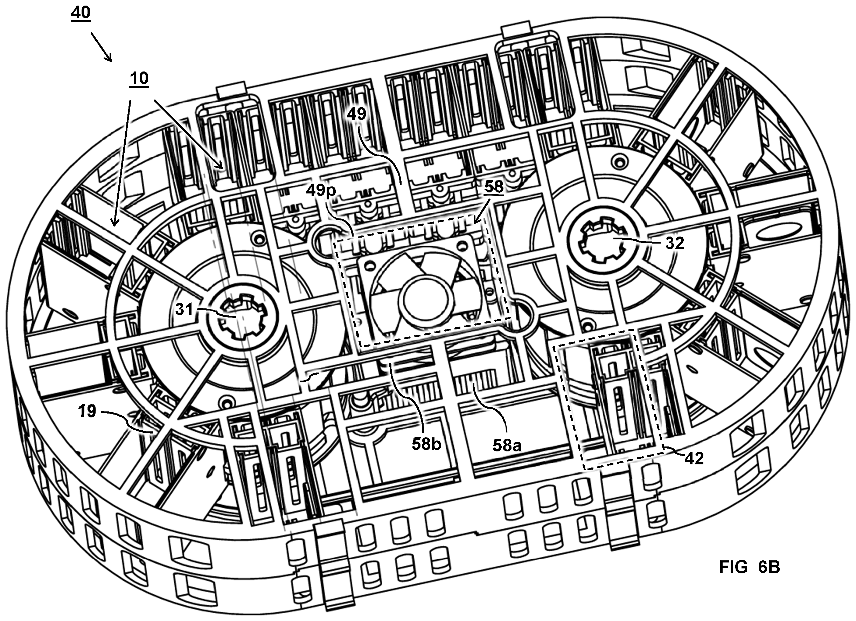

Of course the device can be embodied in numerous different configurations, e.g. having a different number of receptacles, e.g. a different number of storage positions per receptacle, and or a different number of axles, e.g. depending on an intended use, an overall dimensioning, and/or intended dispensing capacity of the device. In a case of 18 links, e.g. as shown in FIG 6B m a number of 72 medicaments or 144 medicament (in case of dual products) can be stored within device for dispensing. Of course, longer or shorter versions can be produced, with correspondingly more or fewer links and therefore higher or lower capacity. For example, the number of axels number can differ, e.g. 1, 2, 3, 4 or more. The number of receptacles can be between 1-100 or more, e.g. 10-50 such as 22 or 40. Likewise a number of storage positions within link can in principle vary over a broad range, e.g. 1-20 or more.

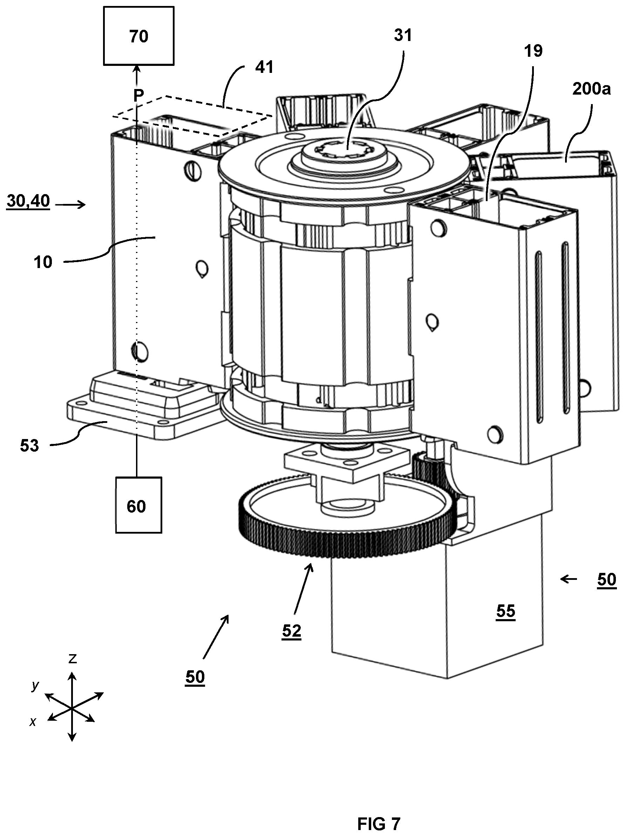

In some embodiments, e.g. as shown, an adapter 19 can be provided. The adapter fits in the receptacle and enables holding of pharmaceutical products having a smaller card dimension. See e.g. Figs 6A, 6B and 7, each depicting receptacles comprising an adapter 19, each adapter configured to accommodate product 200a having a comparatively smaller dimension (note that, for clarity, only a single pharmaceutical product is illustrated).

In some embodiments, e.g. as illustrated in FIG 6A and 6B, the carrousel is embodied within a frame or carrousel housing 49. The frame improves structural integrity and handling of the conveyor. The carrousel together with the optional housing can advantageously be dimensioned so as to provide a comfortable fit within the secure storage compartment of the device, making optimal use of the available space within the secure storage compartment.

To allow dispensing and loading of the products the carrousel comprises one or more loading 41 and/or unloading position 42 providing open access to the guide structures of a receptacle positioned at said position.

Loading can be done manually with the aid of one or more machine loading devices such as cobots. Typically one of the positions is related to a dispensing position of the device. The other can be used as the (or as an additional) loading/unlading position, e.g. within a regulated environment with the cartridge at a filling station outside the secure access storage compartment. Alternatively the carrousel may be loaded while positioned within the device, e.g. with the secure compartment in an open condition.

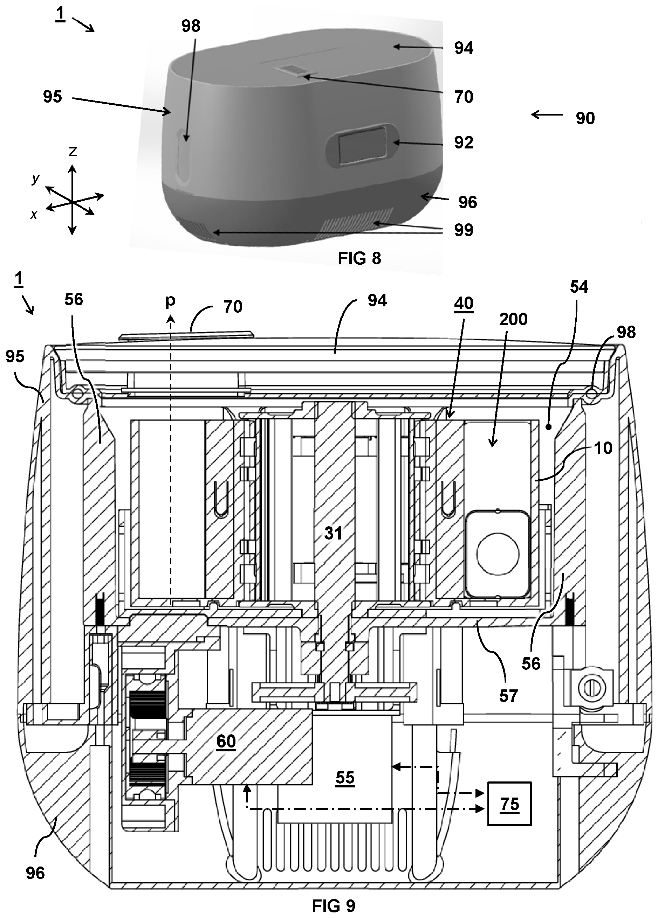

FIG 7 illustrates certain aspects of the carrousel shown in Fig 6. In particular the figure illustrates a positioning system 50 acting on the conveyor 30,40 and configured to position the receptacle 10 to a dispensing position 41 within the storage compartment whereby the pharmaceutical product is positioned between an ejector 60 and a dispensing outlet 70. In the embodiment shown the ejection trajectory P is in an upright position between ejector 60 and dispersion aperture 70. Aspects pertaining to the ejection will be discussed in further detail with reference to FIGs 8-13.

The positioning system comprises a driving element 55 acting on the conveyor to via a drive axle 31. The conveyor can be based on a chain drum or the like to which a number of links has been coupled. The driving element can be any suitable motor. Preferably driving element 55 is controlled by a control system operably connected to the positioning system and the ejector, and configured for logging storage parameters including temperature readings from a temperature sensor housed within the secure access storage compartment. In a preferred embodiment, the driving element 55 comprises a stepper motor. A stepper motor, preferably in configuration with a gearing system 52 can advantageously provide a positioning precision to reliably displace the conveyor such that an edge portion of a card held in storage can positioned along ejection trajectory P for each of the links. Element 53 can comprise means, e.g. optical elements, to align with alignment elements as 18-1,18-2 as provided on the receptacles.

FIG 8 provides a isometric exterior view of a device according the invention in a secured closed state. Depending on a filling the device as shown can be ready to be issued from a pharmacy to an external location for dispensing pharmaceuticals. The main body case can form an exterior housing for the storage compartment and one or more of the auxiliary elements as disclosed herein. Because the device is securely closed unauthorized access to the contents can prevented. As clear from FIGs 8-10 the device can have a lid portion 94 that is covering the storage compartment 54. The lid can be mechanically or electronically secured to the main body 95,96. When unlocked and removed the underlying storage compartment can be accessed. In a preferred embodiment, e.g. as described herein, the controller, the ejector, the positioning system, and the temperature regulation can be predominantly housed within one or more compartments of the main body case at a position below the secure access storage compartment. In a preferred embodiment, e.g. as shown, dispensing outlet 70 is provided within the lid 94.

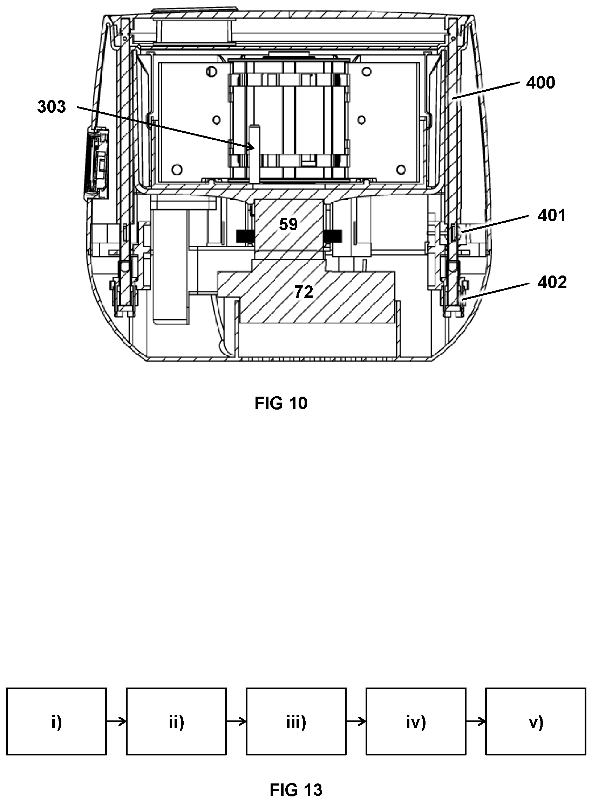

FIG 9 provides a cross-section side view of the device illustrated in FIG 8 along the yz plane and housing a carrousel according to FIG 8 at a position of drive axel 31. As shown carrousel 40 is housed in secure compartment 54 and bound between lid 94 and an inner compartment comprising sidewalls 56 and bottom wall 57. Axels 31 and 32 (also shown in FIG6A, 6B and 7) extend through bottom wall 57 of the secure access compartment. Positioning system 50, including stepper motor 55 as well as ejector 60 and controller 75 are housed in a separate compartment below the carrousel. Lid 94 includes a dispensing outlet having an aperture which extends though the lid. As shown, lid 94 can rest on a shoulder portion connected to outer wall 95. An O-ring 98 extending between seal and the housing mitigates air exchange between ambient and the secure access storage. Removal of the lid and access to the underlying storage compartment can be controlled by one or more locking mechanisms as known in the field. In a preferred embodiment, as best seen in FIG 10, the locking mechanism can comprise a number of limbs 400 coupled to the lid and extending downward towards lock elements 401. To release the lid a spring system 402 can be provided. When the lid is removed storage compartment is accessible, e.g. for filling and/or unloading the receptacle and/to even to entirely replace a carrousel with a pre-filled carrousel.

Insulation material can be provided, e.g. around an external face of walls 56,57 to reduce temperature fluctuations within the storage compartment.

Vents 99, allow for air exchange between ambient and elements housed in a lower compartment of the main body. A display 92, e.g. an e-paper display, can provide visual information or instructions to an end user or care giver. An input interface 98 can be used to activate a dispensing operation. One or more visual, audible, and/or tactile indicators as known in the field can be provided to provide a reminder or a warning. For example, to warn a user that a medication intake is due.

The on demand dispensing can, e.g. be embodied according to a programmed routine operated by the controller. The program can be defined by an end user (e.g. patient) or by a medical caregiver. The program can be defined by a patient. Alternatively, or in addition, dispensing may be according to a pre-programmed routine, set, for example, by a medical professional, such as a pharmacist. Alternatively, or in addition, the dispensing routine may be provided remotely by the pharmacist, medical doctor or otherwise authorized person, for example by directly or wirelessly interfacing with the device or via an intermediate device such as a device or key carried by the patient.

As best seen in FIG 10 the device can advantageously comprise a temperature regulation 59 and/or a humidity regulation (e.g. a humidified/dehumidifier - not indicated). The temperature regulation can advantageously be used to regulate a temperature within the storage compartment within pre-determined bounds. For example, the regulation can be configured to main a temperature in accordance with storage limits for the stored medication. For example, in some embodiments the device can be configured for maintaining a storage temperature in a range of about 2-30°C. More specifically in some embodiments the device can be configured to maintain a storage temperature within a range of 2-8°C or 15-25°C, as appropriate for a category of medication in storage. Appropriate limits can be entered manually or retrieved from a database (e.g. remotely or in a local storage) by the controller in accordance with the prescription or a registered type of medication in held in the receptacles, e.g. within a range of 20-30°C or 0-5 °C or other storage conditions as suitable for the products stored. For traceability purposes the storage conditions can be logged, e.g. to prevent dispensing or restocking of medication when a storage condition is outside a control range.

In a preferred embodiment the temperature regulation comprises a Peltier element. The element can act directly on an outer bound of the compartment, e.g. bottom wall 57. The Peltier technology can be combined with heat pipes and/or a finned block optional fan(s) for controlled air flow.

In some embodiments, bottom 56 and or sidewalls 57 of the inner compartment can be made of a metal. In some embodiments, e.g. as shown in FIG 6B, a heat transfer element 58 may be fitted within an annular space between axels 31 and 32. The heat transfer element can advantageously increase heat transfer to/from the secured access storage space by providing increased surface area, e.g. in the form of fins 58a, optionally in combination with a fan 58b to further improve heat transfer, while being situated at a space between axles 31 and 32. In this case, the bottom and sidewalls of the inner compartment need not be formed of a metal but can advantageously be formed of a plastic composition, e.g. the same composition as exterior walls 95 of the main housing. Forming the bounds of the inner compartment of a plastic composition advantageously reduce heat exchange to/from ambient. Additionally, the inner compartment can be formed monolithically with the main body housing, e.g. walls 95, using conventionally manufacturing methods such as. injection molding. Note that carrousel housing 49 can be provided with an aperture to allow passage 49p of heat transfer element 58.

The temperature regulation can be operably connected to the controller and one or more temperature sensors, including at least one or more inner temperature sensors 303 and one or more optional, but preferable, further sensors for measuring an ambient temperature.

Powering of the electrical components can be performed by conventional means, including but not limited to a mains power connection. In a preferred embodiment, the device comprises a rechargeable battery 72 for powering the device.

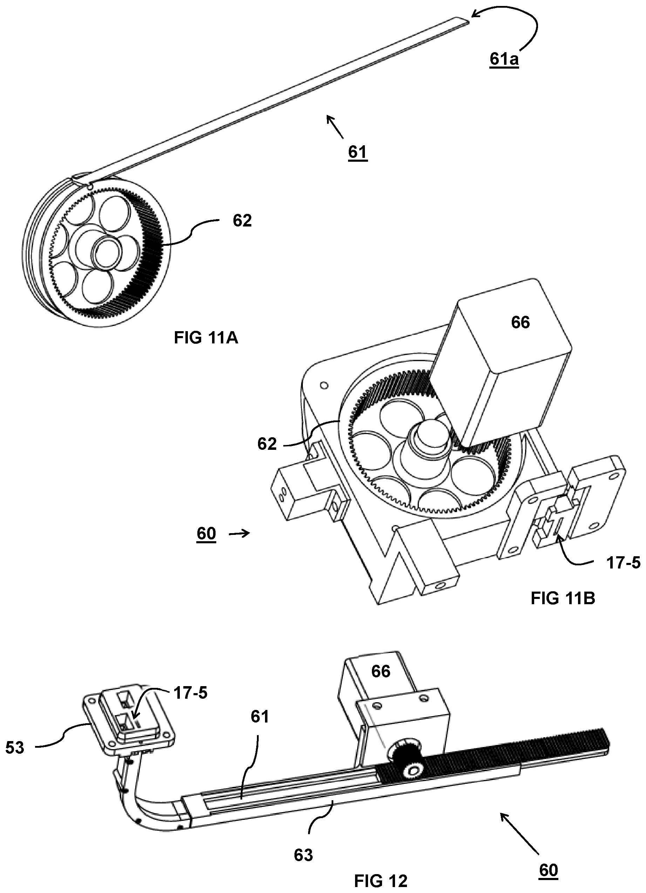

FIGs 11A and 11B illustrate aspects of an ejector of a pharmaceutical dispensing device. FIG 12 illustrates aspects of a different types of ejectors for a pharmaceutical dispensing device. The ejectors comprise an ejector element 61 that is reversibly displaceable along a trajectory between a rest position below the dispensing position when idle and an extended position toward the outlet aperture when activated. The ejector element is preferably an elongate resilient strip 61. The ejectors are configured to engage a bottom end face of the carrier card and thereby push the pharmaceutical product upward from the receptacle and at least partly out of the dispensing outlet for dispensing. A dimension of the end face 61a can be in correspondence with a thickness t_c of the carrier card. For example, the strip can have a thickness ≤ 1 mm.

In some embodiments, e.g. as shown in FIG 9, 11A, 11B the ejector is displaceable by reel assembly 60. The reel can be driven by an electromotor, preferably a stepper motor 66. FIG 11A depicts the ejector element in a fully extended position. In FIG 11B the element is in a retracted position around reel 62.

Alternatively the ejector element 61 can be displaced by a rack-and-pinion assembly 63, e.g. as in FIG 12 depicting a rack and pinion which, similar to reel 60 can be driven by a stepper motor 60.

To guide the strip a guiding element 53, having a guide aperture 17-5 allowing passage of ejector element 11 can be provided at an exit of the ejector.

FIG 13 schematically illustrates a method of dispensing a pharmaceutical product as disclosed herein, preferably the pharmaceutical product as described in relation to FIGs 1-3. The method comprises the steps of i) providing a device, preferably the pharmaceutical dispensing device according to any of claims 1-10. The device comprises at least a secure access storage compartment housing at least one receptacle for holding in storage a pharmaceutical product to be dispensed, means for displacing the pharmaceutical product from one of the at least one receptacle to an outlet for dispensing, and means for logging storage parameters including storage compartment temperature readings.

The method further comprises a step of ii) loading one or more of the at least one receptacle with a pharmaceutical product. Loading can be done in accordance with one or more instructions provided by a medical doctor. A position of loaded medication products can be logged, e.g. by the controller of the device,

The step of loading is generally followed by a iii) securing the secure access storage compartment prior to clearing the device for a dispensing of pharmaceutical products held in storage.

The method further comprises a process of iv) logging the storage parameters, over a period between a time of the clearing the device for the dispensing and a time of receiving the device back from the dispensing. Preferably, the log also includes a loading status of each of the receptacles over time. The log can include a time of dispensing for each of the stored pharmaceutical products over the period specified under iv). Obviously, the method can further comprise steps of logging the outputs of further sensors, when provided. Preferably, the method also comprises regulating a temperature within the secure storage compartment by a temperature regulation unit preferably the temperature regulation as described herein, in dependence of at least a reading of the temperature sensor.

Following a dispensing step, e.g. outside a controlled environment of a pharmacy, the method can advantageously comprise a step of v) restocking any of the pharmaceutical product remaining in storage. Restocking comprising a sub-step of checking whether storage parameters within the storage compartment are within tolerance limits for storing any of the given pharmaceutical products to be restocked.

Steps ii), iii) and iv) can be performed at a pharmacy. Alternatively some or all of these steps can be performed at a different facility operating within tolerance limits for storing the pharmaceutical product, e.g. at a site of medicament manufacturer and/or individualization.

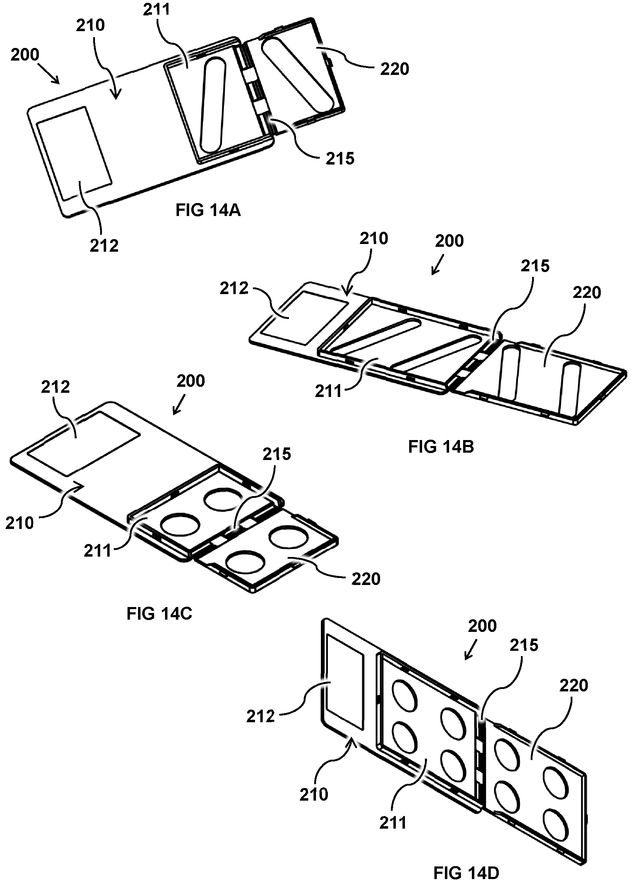

FIGs 14A-D illustrate several exemplary embodiments of a pharmaceutical product 200 that can be used to advantage with the dispensing device as disclosed herein. Each of the illustrated embodiments of the pharmaceutical product 200 comprises a carrier card 210 with a support portion 211 for supporting at least one singulated unit dose package and a data portion 212 for data regarding the unit dose package. In assembled condition, a unit dose blister package is secured between the support portion 211 and a cap 220. The support portion 211 and/or the cap 220 may be provided with one or more cutouts 214, 224 providing access to the unit dose blister package to allow taking individual medicines, e.g. pills, out of the respective blister package(s) without disassembling the pharmaceutical product 200. The size, shape and orientation of the cutouts 214, 224 is preferably adapted to fit the blister package. For example, the embodiment of FIG 14A is arranged for holding a singulated unit dose comprising one blister package, the embodiments of FIG 14B and C are arranged for holding a singulated unit dose comprising two blister packages, and the embodiment of FIG 14D is arranged for holding a singulated unit dose comprising four blister packages. In case the other number of blister packages of a singulated unit dose are to be held, the pharmaceutical product 200 may be adapted accordingly.

As illustrated in FIGs 14A-D, the cap 220 is attached to the carrier card 210 by a living hinge 215 or integral hinge, e.g. a thin flexible hinge (flexure bearing) made from the same material as the carrier card 210 and the cap 220 it connects. The living hinge 215 preferably has a thickness that is relatively thin compared to the thickness of the carrier card and/or cap, thus locally reducing the bending or folding stiffness of the pharmaceutical product to define a hinge axis. For example, the thickness of the living hinge is 5-10x thinner than the thickness of the carrier card 210 and/or cap 220. In preferred embodiments, the living hinge 215 is integrally formed with the carrier card 210 and cap 220. Such an assembly can be manufactured by conventional techniques, such as injection molding or casting. The cutouts 214, 224 in the carrier card and cap can e.g. be formed during the molding or casting process, or can be manufactured in a later stage e.g. by punching or lasering. By having an integral hinge connecting the cap 220 to the carrier card 210, these parts remain together throughout the lifecycle of the pharmaceutical product 200. For example, after manufacturing the pharmaceutical product the cap 220 and corresponding carrier card 210 that are to be assembled together for enclosing a blister package are already physically coupled, which reduces the risk of assembly error of the pharmaceutical product. Also as regards recyclability of the pharmaceutical product it is beneficial when at least the cap is structurally linked to the carrier card 210. The living hinge 215 provides such a structural link. The living hinge 215 may extend between opposing edges of the carrier card and the cap at least partially, e.g. continuously or interrupted over a width of the edges, or may extend between opposing corners of the carrier card and cap.

The unit dose blister package can be secured in the support portion 211 by folding the cap 220 onto the carrier card 210 about the living hinge 215, and subsequently fixating the cap and carrier card to each other. For example, the support portion 211 and/or the cap may be provided with retaining elements arranged for creating a snap fit between the cap and the support portion. For example, each of the cap and the support portion may comprise one or more edges, rims, flanges, hooks, tabs, cams, notches or ribs that cooperatively provide an interference or snap fit between the support portion and the cap when pressed together. Accordingly, there is no need for additional components such as screws or pins, or additional methods such as gluing or welding for fixating the cap and carrier card to each other. For the purpose of clarity and a concise description, features are described herein as part of the same or separate embodiments, however, it will be appreciated that the scope of the invention may include embodiments having combinations of all or some of the features described.

In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise. Where one claim refers to another claim, this may indicate synergetic advantage achieved by the combination of their respective features. But the mere fact that certain measures are recited in mutually different claims does not indicate that a combination of these measures cannot also be used to advantage. The present embodiments may thus include all working combinations of the claims wherein each claim can in principle refer to any preceding claim unless clearly excluded by context.

## Claims

1. A pharmaceutical product (200) configured for use with a pharmaceutical dispensing device, the pharmaceutical product (200) comprising a carrier card (210) having a support portion (211) supporting at least one singulated unit dose blister package (100) and a data portion (212) with regulatory data regarding the unit dose blister package, wherein the carrier card (210) comprises opposing side border portions configured to engage with a receptacle (10) of the pharmaceutical dispensing device, and a bottom end face configured to engage with an ejector element (61) of the pharmaceutical dispensing device, wherein the singulated unit dose blister package (100) is held securely between the support portion (211) and a cap (220), said cap (220) affixed to the support portion (211) from a base around a perimeter of the unit dose blister package (100), **characterized in that** both the support portion and the cap have an aperture (214, 224) allowing access to the blister by pressing on a top film of the blister package (100) to break the bottom seal for releasing the medicament while the unit dose blister package (100) is held securely between the support portion (211) and the cap (220).

2. The pharmaceutical product according to claim 1, wherein the singulated unit dose blister package (100) comprises at least two unit doses packaged in separate blisters in a side by side relation.

3. The pharmaceutical product according to any of the preceding claims, wherein the cap (220) and carrier card (210) are assembled in a click or snap-on relation, preferably by means of a ridge (213) provided on the support region (211) of the carrier card (210) and a corresponding receptacle provided by the cap (220) along its base (221).

4. The pharmaceutical product according to any of claims 1 or 2, wherein the cap (220) and the carrier card (210) are coupled by welding, preferably ultrasonic welding, of parts formed of the same or a similar thermoplastic polymer composition.

5. The pharmaceutical product according to any preceding claim, wherein the cap (220) is attached to the carrier card (210) by a living hinge (215).

6. The pharmaceutical product according to claim 5, wherein the living hinge (215) has a thickness that is 5-10x thinner than a thickness of the carrier card (210).

7. The pharmaceutical product according to claim 5 or 6, wherein the living hinge (215) is integrally formed with the carrier card (210) and cap (220).

8. The pharmaceutical product according to any of claims 5-7, wherein the unit dose blister package is secured in the support portion (211) by folding the cap (220) onto the carrier card (210) about the living hinge (215), and subsequently fixating the cap (220) and carrier card (210) to each other.
